# EUROPÄISCHE PATENTSCHRIFT

(11) **EP 2 640 352 B1**
(45) Veröffentlichungstag und Bekanntmachung des Hinweises auf die Patenterteilung: **30.08.2017**
(21) Anmeldenummer: 11764723.0
(22) Anmeldetag: 29.09.2011
(51) Int. Cl.: A61Q 3/00, A61Q 5/00, A61K 38/39, A23L 33/18, A61K 8/65, A61Q 19/08

(54) **KOLLAGENHYDROLYSAT FÜR DIE VERWENDUNG ZUR VERBESSERUNG DER GESUNDHEIT DER MENSCHLICHEN HAUT, HAARE UND/ODER NÄGEL**
COLLAGEN HYDROLYSATE USED TO IMPROVE THE HEALTH OF HUMAN SKIN, HAIR AND/OR NAILS
HYDROLYSAT DE COLLAGÈNE UTILISÉ POUR AMÉLIORER LA SANTÉ DE LA PEAU, DES CHEVEUX ET/OU DES ONGLES HUMAINS

(30) Priorität: 15.11.2010 DE 102010060564
(43) Veröffentlichungstag der Anmeldung: 25.09.2013
(73) Patentinhaber: Gelita AG, 69412 Eberbach (DE)
(72) Erfinder: HAUSMANNS, Stephan, 68199 Mannheim (DE); GIESEN-WIESE, Monika, 64739 Höchst (DE); OESSER, Steffen, 24960 Glücksburg (DE)
(74) Vertreter: Hoeger, Stellrecht & Partner Patentanwälte mbB
(86) Internationale Anmeldenummer: PCT/EP2011/067028
(87) Internationale Veröffentlichungsnummer: WO 2012/065782

(56) Entgegenhaltungen:
- EP-A1- 0 120 722
- CN-A- 101 518 645
- DE-A1- 4 244 418
- FR-A1- 2 900 155
- US-A- 4 130 555
- HUO J X ET AL: "Study on Enzymatic Hydrolysis of Gadus morrhua Skin Collagen and Molecular Weight Distribution of Hydrolysates", AGRICULTURAL SCIENCES IN CHINA,, Bd. 8, Nr. 6, 1. Juni 2009 (2009-06-01), Seiten 723-729, XP026221114, ISSN: 1671-2927, DOI: 10.1016/S1671-2927(08)60271-0 [gefunden am 2009-06-01]

## Beschreibung

Die vorliegende Erfindung betrifft die Verwendung von Kollagenhydrolysat zur Stimulation der Biosynthese extrazellulärer Matrixproteine durch Hautzellen, zur Erhöhung der Spannkraft der Haut, zur Verminderung der Faltenbildung und/oder zur Erhöhung des Feuchtigkeitsgehalts der Haut.

Es ist seit einiger Zeit bekannt, dass durch die orale Aufnahme von Kollagenhydrolysat vorteilhafte Effekte in Bezug auf die Gesundheit der Haut, aber auch der Haare und/oder Nägel beim Menschen erzielt werden können (siehe z.B. Vivian Zague: "A new view concerning the effects of collagen hydrolysate intake on skin properties" in Arch. Dermatol. Res. 2008 (9) 479-483). Unter einer Verbesserung der Gesundheit der Haut wird in diesem Zusammenhang jede positive Beeinflussung der natürlichen Eigenschaften und Funktionen der Haut verstanden, wobei diese Eigenschaften und Funktionen sowohl in Folge der Alterung als auch zusätzlich durch verschiedene negative Umwelteinflüsse beeinträchtigt werden können. Auch auf die Eigenschaften der Haare und/oder Nägel wirkt sich die Aufnahme von Kollagenhydrolysat vorteilhaft aus.

Die US 4,130,555 offenbart eine Peptidmischung, die durch Hydrolyse eines Kollagen enthaltenden Materials oder Gelatine hergestellt ist, und die eine relativ enge Molekulargewichtsverteilung aufweist.

Die DE 42 44 418 A1 offenbart Peptid-Präparate und Verfahren zu deren Herstellung, vorzugsweise durch partielle Hydrolyse von Bindegewebe, Kollagen, Elastin und Keratin, die Tripeptide oder Peptidsequenzen Gly-His-Lys und/oder Gly-Asp-Ser enthalten.

Die EP 0 120 722 A1 offenbart ein Verfahren für die Stimulation der Vermehrung von Zellen, insbesondere von Fibroblasten, durch die Wirkung von Peptiden oder Polypeptiden, wobei die Peptide oder Polypeptide in einer hydratisierten Lipidphase verkapselt werden.

Die FR 2 900 155 A1 offenbart ein Hydrolysat aus Knorpel mit einem Anteil von 45 bis 70 Gew.% an hydrolysiertem Kollagen vom Typ II, wobei das mittlere Molekulargewicht der Peptidfraktion zwischen 500 und 1.000 Dalton liegt.

In einem Artikel von J. Huo und Z. Zhao in Agricultural Sciences in China 2009 (8) 723-729 wird die enzymatische Hydrolyse von Kollagen aus der Haut von Kabeljau *(Gadus morrhua)* sowie dessen Molekulargewichtsverteilung beschrieben.

Die CN 101 518 645 offenbart ein Produkt aus marinen Kollagenpeptiden und dessen therapeutische Anwendung für die Verbesserung der Nierenfunktion.

Die Broschüre "Peptan - The perfect protein for a healthy lifestyle" der Firma Rousselot beschreibt Hydrolysate aus Kollagen von Schweinen, Rindern und Fischen und deren vorteilhafte Wirkung auf das Erscheinungsbild der Haut.

In einem Artikel von G. Zhang et al. in Food Hydrocolloids 2009 (23) 2001-2007 wird die Untersuchung von Markerpeptiden in Kollagenhydrolysaten, die durch Hydrolyse von boviner und porciner Gelatine mit Trypsin erhalten wurden, beschrieben.

Der Erfindung liegt die Aufgabe zugrunde, die positiven Effekte von Kollagenhydrolysat auf die Gesundheit von Haut, Haaren und/oder Nägeln weiter zu steigern und ein für diese Verwendung besonders effektives Kollagenhydrolysat vorzuschlagen.

Diese Aufgabe wird durch die nicht-therapeutische Verwendung eines Kollagenhydrolysats gemäß Anspruch 1 gelöst.

Kollagenhydrolysate enthalten Peptide mit unterschiedlichen Kettenlängen bzw. Molekulargewichten, die bei der Spaltung der Proteinketten des Kollagens entstehen, wobei sich die Molekulargewichtsverteilungen dieser Peptide in Abhängigkeit von den Herstellungsbedingungen des Hydrolysats deutlich unterscheiden können. Es hat sich nun überraschenderweise gezeigt, dass sich ein Kollagenhydrolysat mit den vorstehend genannten Eigenschaften in verschiedener Hinsicht besonders vorteilhaft auf die Hautgesundheit auswirkt, d.h. deutlich bessere Ergebnisse zeigt als die bisher eingesetzten Kollagenhydrolysate, die einen wesentlich geringeren Anteil an niedermolekularen Peptiden enthalten und/oder die die charakteristischen Peptide nicht enthalten.

Die Molekulargewichtsverteilung des Kollagenhydrolysats kann z.B. mittels einer Gelpermeationschromatographie unter Verwendung eines Kalibrierungsstandards aus definierten Kollagenfragmenten sehr genau und reproduzierbar bestimmt werden.

Mindestens 45 Gew.% des Kollagenhydrolysats weisen ein Molekulargewicht von weniger als 1.500 Da auf, d.h. ein gewisser Anteil der Peptide des Kollagenhydrolysats ist besonders kurzkettig. Es hat sich gezeigt, dass durch solche besonders niedermolekularen Anteile, die in derzeit verwendeten Kollagenhydrolysaten nur in wesentlich geringerer Menge enthalten sind, sehr ausgeprägte Effekte auf die Hautgesundheit erzielt werden können.

Das mittlere Molekulargewicht (Gewichtsmittel M_{W}) des erfindungsgemäß verwendeten Kollagenhydrolysats liegt typischerweise im Bereich von ca. 1.700 bis ca. 2.300 Da.

Das Vorhandensein der charakteristischen Peptide des Kollagenhydrolysats, die interessanterweise wesentlich zu dessen Wirksamkeit beitragen, kann insbesondere mittels MALDI-Massenspektroskopie bestimmt werden, wobei die charakteristischen Peptide im Massenspektrum als Peaks auftreten. Die mindestens vier charakteristischen Peptide weisen in einer mittels MALDI-Massenspektroskopie bestimmten Molekulargewichtsverteilung eine mindestens doppelte Intensität, weiter bevorzugt eine mindestens vierfache Intensität im Vergleich zu ihrer Umgebung auf.

Bei einer bevorzugten Ausführungsform der Erfindung umfasst das Kollagenhydrolysat ein Peptid zwischen 620 und 690 Da, ein Peptid zwischen 790 und 860 Da, ein Peptid zwischen 980 und 1.050 Da und ein Peptid zwischen 1.175 und 1.245 Da auf. Diese Peptide unterscheiden das Kollagenhydrolysat gemäß der Erfindung auf charakteristische Weise von bekannten Kollagenhydrolysaten.

Das Kollagenhydrolysat kann zusätzlich auch charakteristische Peptide zwischen 1.500 und 3.500 Da aufweisen, die ebenfalls ein Unterscheidungsmerkmal zu Kollagenhydrolysaten gemäß dem Stand der Technik darstellen.

Bevorzugt weist das Kollagenhydrolysat einen Anteil an Hydroxyprolin von 12 Gew.% oder mehr auf. Die durch post-translationale Hydroxylierung von Prolin gebildete Aminosäure Hydroxyprolin kommt ausschließlich in Kollagen vor, sodass ein hoher Anteil an Hydroxyprolin im Kollagenhydrolysat ein Maß für die weitgehende Abwesenheit von anderen Bindegewebsproteinen (z.B. Elastin und Proteoglykane) darstellt, deren Fragmente in Abhängigkeit vom Herstellungsverfahren in gewissen Mengen ebenfalls in Kollagenhydrolysaten enthalten sein können.

Günstig ist es, wenn das Kollagenhydrolysat durch enzymatische Hydrolyse von Gelatine hergestellt ist. Gelatine umfasst denaturiertes Kollagen und wird mittels verschiedener, dem Fachmann bekannter Verfahren aus dem Bindegewebe oder aus den Knochen verschiedener Tierarten gewonnen. Im Rahmen der vorliegenden Erfindung wird die als Ausgangsmaterial für das Kollagenhydrolysat eingesetzte Gelatine vorzugsweise aus der Haut von Säugetieren, insbesondere aus Schweineschwarte oder Rinderspalt, gewonnen, wobei aber auch die Verwendung von Gelatine aus Geflügel nicht ausgeschlossen ist.

Die enzymatische Hydrolyse der Gelatine erfolgt durch mindestens eine Endoprotease, wobei es im Rahmen der Erfindung vorgesehen ist, mehrere Endoproteasen (d.h. mindestens zwei verschiedene Endoproteasen) einzusetzen, um dadurch das Aminosäureprofil des resultierenden Kollagenhydrolysats entsprechend zu beeinflussen und die positive Wirkung des Hydrolysats auf die Gesundheit von Haut, Haaren und/oder Nägeln zu erhöhen.

Gemäß der Erfindung ist das Kollagenhydrolysat durch die nacheinander folgende Einwirkung von mindestens zwei Endoproteasen mit einer unterschiedlichen Spezifität, nämlich von mindestens zwei verschiedenen Metalloproteasen und/oder Serinproteasen, hergestellt, d.h. von Proteasen, die die Aminosäuresequenz der Kollagenmoleküle jeweils vor bzw. hinter bestimmten Aminosäuren spalten. Es handelt es sich bei den Metalloproteasen und/oder Serinproteasen um Enzyme aus den Mikroorganismen *Bacillus subtilis* oder *Bacillus amyloliquefaciens* bzw. aus *Bacillus licheniformis.*

Durch die Auswahl geeigneter Endoproteasen kann nicht nur die charakteristische Molekulargewichtsverteilung des Kollagenhydrolysats erhalten werden, sondern es wird auch die Art der Aminosäuren an den Termini der in dem Hydrolysat enthaltenen Peptide beeinflusst. In dieser Hinsicht ist es z.B. bevorzugt, wenn mindestens 50% der N-terminalen Aminosäuren des Kollagenhydrolysats hydrophobe Aminosäuren sind, insbesondere Alanin, Leucin und Isoleucin.

Die Erfindung betrifft insbesondere auch die nicht-therapeutische Verwendung des Kollagenhydrolysats zur Stimulation der Biosynthese extrazellulärer Matrixproteine durch Hautzellen. Hautzellen umfassen insbesondere Fibroblasten, die u.a. Kollagen (hauptsächlich vom Typ I), Elastin sowie Proteoglykane synthetisieren. Die Bildung dieser Proteine in ausreichender Menge ist entscheidend für den Aufbau bzw. die Regeneration der extrazellulären Matrix der Haut, die wiederum wesentlich bestimmend ist für die Eigenschaften der Haut wie Spannkraft und Elastizität sowie ihren Feuchtigkeitshaushalt.

Zu den Hautzellen zählen auch die Keratinozyten, die sowohl für die Verhornung der äußersten Hautschicht als auch für die Bildung von Haaren und Nägeln verantwortlich sind. Somit kann eine Stimulation dieser Zellen durch das erfindungsgemäß verwendete Kollagenhydrolysat eine Verbesserung der Barrierefunktion der Haut sowie der Gesundheit der Haare und/oder Nägel bewirken.

Ein wesentlicher Aspekt der Erfindung betrifft die nicht-therapeutische Verwendung des Kollagenhydrolysats zur Erhöhung der Spannkraft der Haut und/oder der Verminderung der Faltenbildung. Diese Eigenschaften der Haut verschlechtern sich in der Regel alterungsbedingt sowie in Folge von Umwelteinflüssen wie z.B. UV-Strahlung oder toxischen Substanzen. Da für die Spannkraft und Elastizität der Haut insbesondere die Matrixproteine Kollagen und Elastin verantwortlich sind, kann durch deren vermehrte Synthese, die durch das erfindungsgemäß verwendete Kollagenhydrolysat in besonderem Maße stimuliert wird, diesen Effekten entgegengewirkt und die Hautgesundheit deutlich verbessert werden.

Ein weiterer wichtiger Aspekt der Erfindung betrifft die nicht-therapeutische Verwendung des Kollagenhydrolysats zur Erhöhung des Feuchtigkeitsgehalts der Haut. Einen wesentlichen Beitrag zur Fähigkeit der Haut, ausreichende Mengen an Feuchtigkeit zu binden, leisten die in der extrazellulären Matrix enthaltenen Proteoglykane (z.B. Versican, Biglykan und Decorin), deren Synthese durch das erfindungsgemäß verwendete Kollagenhydrolysat ebenfalls nachweislich stimuliert wird. Auch der Feuchtigkeitsgehalt der Haut kann durch schädliche Umwelteinflüsse wie UV-Strahlung reduziert sein, wobei der Feuchtigkeitsverlust häufig mit einer geringeren Geschmeidigkeit, Rissigkeit und überschüssigen Verhornung der Haut einhergeht.

Ein weiterer Aspekt der Erfindung betrifft das Kollagenhydrolysat für die therapeutische Verwendung zur Verbesserung der Barrierefunktion der Haut. Eine wesentliche Rolle für diese Barrierefunktion spielen die so genannten CE-Proteine ("Cornified Envelope"-Proteine), u.a. Involucrin, Loricrin und Filaggrin, deren Biosynthese
durch das niedermolekulare Kollagenhydrolysat nachweislich stimuliert wird. Somit wird durch die erfindungsgemäße Verwendung von Kollagenhydrolysat die Verminderung des Feuchtigkeitsverlustes, die Barrierefunktion sowie der natürliche Schutz der Haut gegen pathogene Keime und toxische Substanzen verbessert.

Die erfindungsgemäße Verwendung des Kollagenhydrolysats führt zusätzlich auch zu einer anti-oxidativen Wirkung im Bereich der Haut, wodurch die Häufigkeit von DNA-Schädigungen (Mutationen), z.B. durch UV-Strahlung oder mutagene Substanzen, reduziert werden kann. Solche Mutationen stellen eine der Ursachen für die frühzeitige Alterung von Zellen dar und fördern dadurch Alterungserscheinungen der Haut (Nachlassen der Spannkraft, Faltenbildung usw.), sodass dieser Vorgang durch die erfindungsgemäße Verwendung gehemmt werden kann.

Gemäß einer bevorzugten Ausführungsform der Erfindung ist das Kollagenhydrolysat für eine orale Aufnahme vorgesehen. Bei oraler Aufnahme ergibt sich häufig ein effektiverer Transport des Kollagenhydrolysats über den Blutkreislauf zum Wirkungsort, d.h. insbesondere zu den Hautzellen, als bei einer topischen Anwendung. Zudem ist diese Anwendungsform für den Benutzer in der Regel mit einem wesentlich geringeren Aufwand verbunden.

Vorzugsweise wird das Kollagenhydrolysat als Nahrungsergänzungsmittel eingesetzt. Das Kollagenhydrolysat dient dabei in der Regel der Verbesserung der Hautgesundheit im Sinne einer allgemeinen Gesundheitsvorsorge oder auch einer kosmetischen Anwendung; solche Nahrungsergänzungsmittel können als "Nutraceuticals" oder "Nutricosmetics" bezeichnet werden. Aber auch eine Verwendung des Kollagenhydrolysats zur Behandlung eines klinisches Krankheitsbildes ist im Rahmen der vorliegenden Erfindung möglich, so z.B. bei Vorliegen einer atopischen Dermatitis, bei der die Patienten eine erhöhte Neigung zu trockener, rissiger Haut haben.

Das Nahrungsergänzungsmittel kann in nahezu beliebiger Form angeboten werden, z.B. in Form von Tabletten, Kapseln, Dragees, Pastillen oder auch einer Lösung (z.B. in Einzelampullen oder in Getränken).

Das Kollagenhydrolysat kann alternativ auch in einem Nahrungs- oder Genussmittel enthalten sein, z.B. in Süßwaren oder in einem Instantpulver zur Zubereitung von Getränken. Das Hydrolysat kann auf diese Weise vom Benutzer ohne zusätzlichen Aufwand im Rahmen der normalen Ernährung aufgenommen werden (so genanntes "Functional Ford"). In diesem Zusammenhang ist es besonders vorteilhaft, wenn das Kollagenhydrolysat im Wesentlichen geschmacksneutral ist.

Günstig ist es, wenn eine täglich Aufnahme von ca. 1,5 bis 5 g, bevorzugt ca. 2 bis 3 g, weiter bevorzugt ca. 2,3 bis 2,7 g des Kollagenhydrolysats vorgesehen ist. Es hat sich gezeigt, dass durch die orale Aufnahme dieser Menge an Hydrolysat bereits innerhalb von wenigen Wochen die Hautgesundheit merklich verbessert werden kann.

Eine weitere Ausführungsform der Erfindung betrifft die topische Anwendung des Kollagenhydrolysats, d.h. die Anwendung in Form von kosmetischen Produkten zum Aufbringen auf die Haut, die Haare und/oder die Nägel. Das Kollagenhydrolysat kann dabei insbesondere in einer Creme, einer Salbe, einer Lotion oder einem Gel enthalten sein. Günstig ist es auch, wenn das Kollagenhydrolysat einem Körperpflegeprodukt wie z.B. einem Duschgel oder einem Haarshampoo beigefügt wird, beispielsweise in einer Menge von ca. 5 bis 10 Gew.%.

Diese und weitere Vorteile der Erfindung werden anhand der nachfolgenden Beispiele unter Bezugnahme auf die Figuren näher beschrieben. Es zeigen im Einzelnen:
- Figuren 1A bis 1C:: Diagramme betreffend die Stimulation der Synthese von Typ-I-Kollagen, Biglykan bzw. Versican;
- Figuren 2A und 2B:: Diagramme betreffend die Erhöhung der Hautfeuchtigkeit bei haarlosen Mäusen;
- Figur 3:: ein Diagramm betreffend die Stimulation der Synthese von CE-Proteinen;
- Figur 4A bis 4C:: MALDI-Massenspektren verschiedener Kollagenhydrolysate; und
- Figur 5A und 5B:: Diagramme betreffend die Stimulation der Synthese von Typ-I-Kollagen, Decorin und Versican.

### Beispiele

### 1. Herstellung und Eigenschaften des Kollagenhydrolysats

Zur Herstellung eines erfindungsgemäßen Kollagenhydrolysats wird als Ausgangsmaterial eine wässrige Lösung einer Schweineschwartengelatine (Typ A, 200 bis 250 g Bloom) mit einer Konzentration von 20 bis 40 Gew.% (Trockensubstanz) eingesetzt. Die Gelatine wird durch die nacheinander folgende Einwirkung von zwei verschiedenen Endoproteasen mikrobiellen Ursprungs bei 50 bis 60°C während 120 bis 180 min enzymatisch hydrolysiert, wobei als erstes Enzym eine Endoprotease aus *Bacillus subtilis* oder aus *Bacillus amyloliquefaciens* verwendet wird und als zweites Enzym eine Endoprotease aus *Bacillus licheniformis.* Anschließend werden die Enzyme thermisch inaktiviert und die Lösung sprühgetrocknet.

Die Molekulargewichtsverteilung des resultierenden Kollagenhydrolysats kann mittels Gelpermeationschromatographie unter Verwendung der folgenden Parameter bestimmt werden:

| | |
|---|---|
| Stationäre Phase: | TSK 2000 SW XL (Tosoh Bioscience GmbH) |
| Mobile Phase: | 0,4 mol/l Natriumdihydrogenphosphat pH 5,3 |
| Flussrate: | 0,5 ml/min |
| Kalibrierungsstandard: | definierte Kollagen-Typ-I-Fragmente (FILK, Freiberg) |
| Detektion: | UV-Detektor Knauer K-2501 bei 214 nm |

Die Bestimmung ergab eine Molekulargewichtsverteilung des erfindungsgemäßen Kollagenhydrolysats (im Folgenden als niedermolekulares Hydrolysat bezeichnet) gemäß der nachfolgenden Tabelle 1. Zum Vergleich ist in der Tabelle 1 auch die Molekulargewichtsverteilung eines kommerziell erhältlichen Kollagenhydrolysats angegeben, die mit derselben Methode bestimmt wurde (im Folgenden als hochmolekulares Hydrolysat bezeichnet):

**Tabelle 1**

| MW-Bereich | niedermol. Hydrolysat | hochmol. Hydrolysat |
|---|---|---|
| > 7.500 Da | < 5 Gew.% | < 10 Gew.% |
| 3.500 - 7.500 Da | ca. 12-18 Gew.% | ca. 25-35 Gew.% |
| 1.500 - 3.500 Da | ca. 25-31 Gew.% | ca. 29-35 Gew.% |
| 500 - 1.500 Da | ca. 40-46 Gew.% | ca. 24-30 Gew.% |
| < 500 Da | ca. 5-10 Gew.% | ca. 2-5 Gew.% |

Der Hydroxyprolingehalt dieses niedermolekularen Hydrolysats beträgt ca. 12 bis 13 Gew.% und kann nach Oxidation mit Chloramin-T und Umsetzung mit p-Dimethylamino-benzaldehyd photometrisch bestimmt werden. Mehr als 50% der N-terminalen Aminosäuren des Hydrolysats sind hydrophobe Aminosäuren, insbesondere Alanin, Leucin und Isoleucin.

### 2. Stimulation der Synthese von extrazellulären Matrixproteinen

Die Stimulation der Synthese von Kollagen (Typ I) sowie der Proteoglykane Biglykan und Versican wurde *in vitro* an humanen dermalen Fibroblasten (Hautzellen) untersucht. Hierfür wurden die Zellen für 24 Stunden mit jeweils 0,5 mg/ml des niedermolekularen bzw. des hochmolekularen Hydrolysats inkubiert und anschließend die Expression von Kollagen-RNA, Biglykan-RNA und Versican-RNA mittels Realtime-PCR bestimmt und semiquantitativ (bezogen auf eine Kontrolle ohne Hydrolysat) ausgewertet.

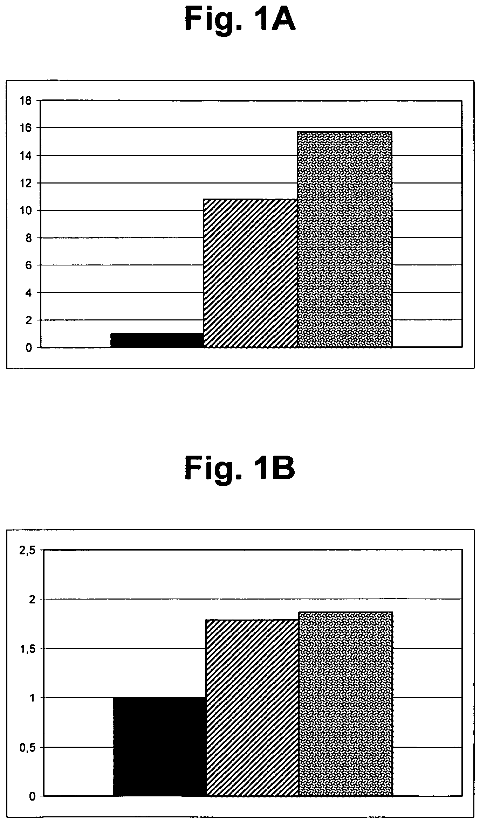

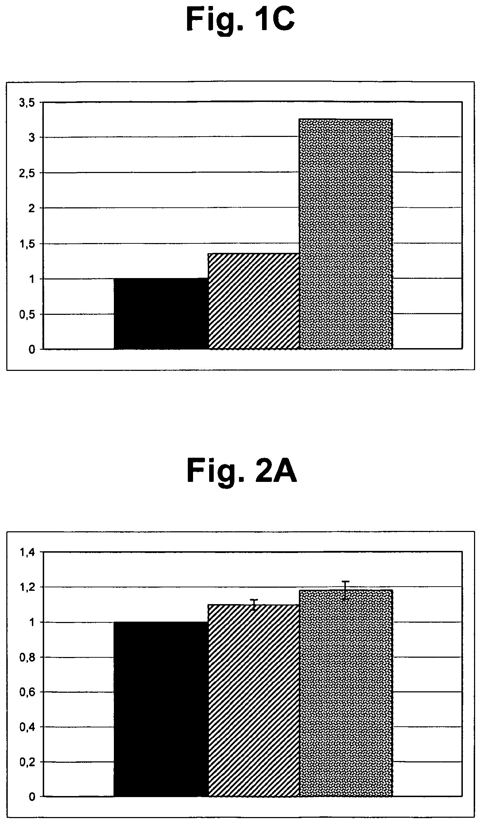

Die Ergebnisse sind als Säulendiagramme für Typ-I-Kollagen in der Figur 1A, für Biglykan in der Figur 1B und für Versican in der Figur 1C dargestellt, wobei die Diagramme jeweils den Mittelwert aus mindestens 18 Messungen zeigen. Auf der Abszisse ist die RNA-Expression relativ zur Kontrolle (=1) aufgetragen. Die linke ausgefüllte Säule steht jeweils für die Kontrolle, die mittlere schraffierte Säule für das hochmolekulare Hydrolysat (als Vergleich) und die rechte gepunktete Säule für das niedermolekulare Hydrolysat (gemäß der Erfindung).

Es zeigt sich, dass die Synthese aller drei Matrixproteine durch beide Kollagenhydrolysate stimuliert wird, wobei der positive Effekt des niedermolekularen Hydrolysats jeweils stärker ausgeprägt ist als derjenige des hochmolekularen Hydrolysats. Beim Kollagen, welches neben Elastin hauptverantwortlich für die Spannkraft und Elastizität der Haut ist, sowie beim Versican, das für den Feuchtigkeitshaushalt der Haut eine wichtige Rolle spielt, fällt der verstärkte Effekt des niedermolekularen Hydrolysats besonders deutlich aus.

### 3. Erhöhung des Feuchtigkeitsgehalts der Haut

Die Beeinflussung der Hautfeuchtigkeit durch Kollagenhydrolysat wurde an haarlosen Mäusen direkt untersucht. Haarlose Mäuse stellen ein etabliertes Modellsystem dar, das bei dermatologischen Fragestellungen häufig eingesetzt wird, und die daraus gewonnenen Erkenntnisse sind prinzipiell auf die menschliche Haut übertragbar (siehe z.B. T. Fujimura et al.; J. Dermatol. Sci. 2000 (24) 105-111 und Y. Nishimori et al.; J. Invest. Dermatol. 2001 (117) 1458-1463).

Die Tiere wurden während eines Zeitraums von drei Wochen täglich mit 150 µg Kollagenhydrolysat pro kg Körpergewicht gefüttert, die Kontrollgruppe erhielt stattdessen BSA. Gleichzeitig erhielten alle Tiere eine wöchentliche UV-B-Strahlungsdosis von 18 mJ/cm² Hautoberfläche, wodurch die Hautfeuchtigkeit negativ beeinflusst wird.

Der Feuchtigkeitsgehalt der Haut wurde nach einer Woche und nach drei Wochen mit einem Corneometer CM 825 (Hersteller: Courage & Khazaka) gemessen. Das Messprinzip beruht hierbei auf der Änderung der Kapazität eines Messkondensators durch die Dielektrizitätskonstante des in den oberen Hautschichten gebundenen Wassers, die sich von den Dielektrizitätskonstanten der meisten anderen Stoffe deutlich unterscheidet.

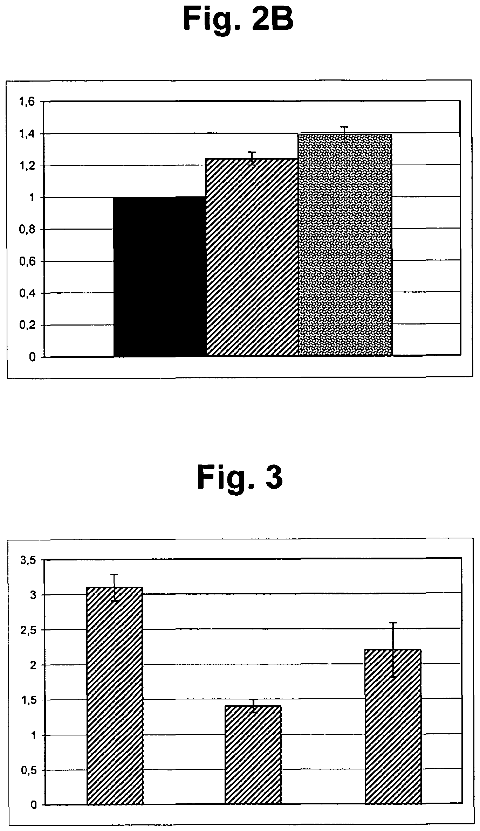

Die Ergebnisse sind als Säulendiagramme für die Messung nach einer Woche in der Figur 2A und für die Messung nach drei Wochen in der Figur 2B dargestellt, wobei die Diagramme jeweils den Mittelwert und den Standardfehler aus 7 Messungen zeigen. Auf der Abszisse ist die Hautfeuchtigkeit relativ zur Kontrolle (=1) aufgetragen. Die linke ausgefüllte Säule steht jeweils für die Kontrolle, die mittlere schraffierte Säule für das hochmolekulare Hydrolysat (als Vergleich) und die rechte gepunktete Säule für das niedermolekulare Hydrolysat (gemäß der Erfindung).

Es zeigt sich, dass die Erhöhung der Hautfeuchtigkeit durch das niedermolekulare Hydrolysat sowohl nach einer Woche als auch nach drei Wochen jeweils stärker ist als durch das hochmolekulare Hydrolysat. Dies ist ein weitere Beleg für die besondere Wirksamkeit des erfindungsgemäß verwendeten Hydrolysats bei der Verbesserung der Hautgesundheit.

### 4. Stimulation der Synthese von CE-Proteinen

So genannte "Cornified Envelope"-Proteine spielen eine wichtige Rolle für die Barrierefunktion der Haut gegen das Eindringen von pathogenen Keimen und toxischen Substanzen. Die Synthese der CE-Proteine Involucrin, Loricrin und Filaggrin wurde bei haarlosen Mäusen bestimmt, die zuvor fünf Wochen mit täglich 150 µg Kollagenhydrolysat pro kg Körpergewicht gefüttert wurden (wie oben beschrieben). Die Quantifizierung der Proteine relativ zu einer Kontrollgruppe (Fütterung mit BSA) erfolgte mittels SDS-Polyacrylamid-Gelelektrophorese und Western-Blot mit spezifischen Antikörpern nach Extraktion der Proteine aus der Haut.

Die Ergebnisse sind als Säulendiagramm in der Figur 3 dargestellt, wobei das Diagramm jeweils den Mittelwert und den Standardfehler aus 7 Messungen zeigt. Auf der Abszisse ist die Menge der CE-Proteine nach Fütterung mit dem niedermolekularen Hydrolysat relativ zur Kontrolle (=1) aufgetragen. Die linke Säule steht für Involucrin, die mittlere Säule für Loricrin und die rechte Säule für Filaggrin.

Es zeigt sich, das die Synthese aller drei untersuchten CE-Proteine durch die orale Aufnahme des erfindungsgemäß verwendeten Kollagenhydrolysats stimuliert wird, im Fall von Involucrin sogar um mehr als das Dreifache.

### 5. Anti-oxiadtive Wirkung des Kollagenhydrolysats

Die anti-oxidative Wirkung des niedermolekularen Kollagenhydrolysats wurde in einem zellfreien System mittels eine Malondialdehyd-Assays ermittelt.

Dabei zeigte sich, dass die Bildung von reaktiven Sauerstoff-Spezies durch das Kollagenhydrolysat um durchschnittlich ca. 7% verringert wird. Im Bereich der Haut kann dadurch die Häufigkeit von DNA-Schädigungen reduziert werden, sodass Alterungserscheinungen durch die erfindungsgemäße Verwendung entgegengewirkt werden kann.

### 6. Analyse der Molekulargewichtsverteilung mittels MALDI-MS

Das gemäß Beispiel 1 hergestellte, niedermolekulare Kollagenhydrolysat gemäß der Erfindung, das ein mittleres Molekulargewicht von ca. 2.000 Da aufweist (im folgenden Hydrolysat A), wurde zwei kommerziell erhältlichen Kollagenhydrolysaten mit einem mittleren Molekulargewicht von ca. 2.100 Da (im Folgenden Hydrolysat B) und ca. 2.900 Da (im Folgenden Hydrolysat C) gegenübergestellt.

Die genauen Molekulargewichtsverteilungen dieser drei Hydrolysate wurden mittels MALDI-Massenspektroskopie (MALDI-MS) analysiert. Hierzu wurden die Proben in 0,1%iger Trifluoressigsäure auf eine Endkonzentration von 10 µg/µl eingestellt und anschließend unter Verwendung von µC₁₈-Material gereinigt. Die Proben wurden mit einer HCCA-Matrix auf ein MALDI-Target präpariert und die Massenspektren unter Verwendung eines Ultraflex-III-TOF/TOF-Massenspektrometers (Hersteller: Bruker Daltonics) bestimmt.

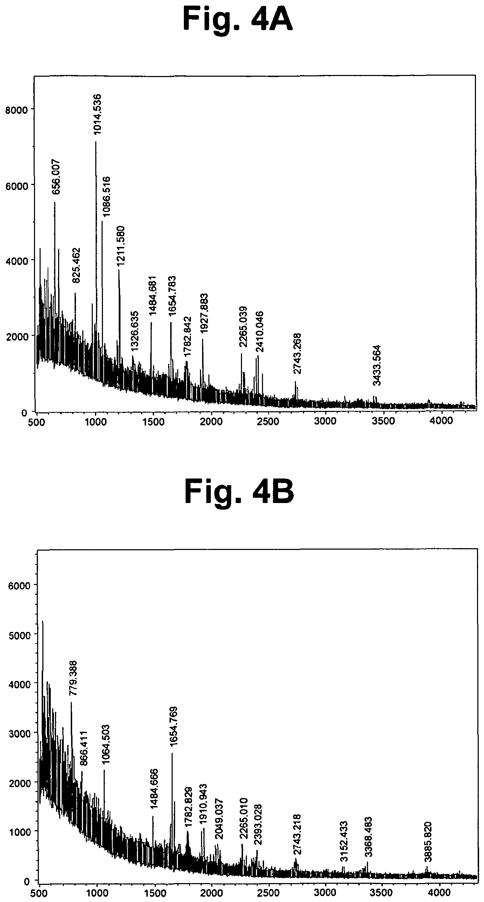

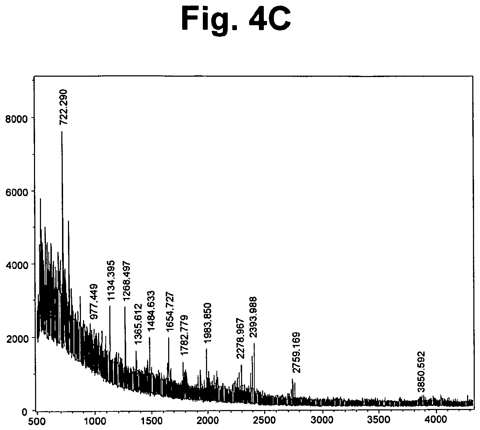

Die Figuren 4A bis 4C zeigen die entsprechenden Massenspektren bzw. Molekulargewichtsverteilungen der Kollagenhydrolysate A, B bzw. C, wobei auf der Ordinate das Molekulargewicht bzw. die Massenzahl und auf der Abszisse die Intensität aufgetragen ist. Ein Vergleich der drei Spektren zeigt, dass das erfindungsgemäße Hydrolysat A die folgenden charakteristischen Peptide gemäß der Tabelle 2 umfasst, wobei die entsprechenden Peaks eine doppelte bis vierfache Intensität im Vergleich zu ihrer Umgebung aufweisen:

**Tabelle 2**

| |
|---|
| ca. 656 Da |
| ca. 825 Da |
| ca. 1.014 Da |
| ca. 1.211 Da |
| ca. 1.927 Da |
| ca. 2.410 Da |
| ca. 3.433 Da |

Insbesondere die vier Peptide zwischen 600 und 1.500 Da haben keine Entsprechungen bei den beiden kommerziellen Hydrolysaten B und C, und sind somit für das Hydrolysat A besonders charakteristisch.

### 7. Stimulation der Synthese von extrazellulären Matrixproteinen

Die Stimulation der Synthese von Kollagen (Typ I) sowie der Proteoglykane Decorin und Versican wurde *in vitro* an humanen dermalen Fibroblasten (Hautzellen) untersucht. Hierfür wurden die Zellen für 24 Stunden mit jeweils 0,5 mg/ml der Hydrolysate A, B bzw. C inkubiert und anschließend die Expression von Kollagen-RNA, Decorin-RNA und Versican-RNA mittels Realtime-PCR bestimmt und semiquantitativ ausgewertet. Decorin spielt eine wichtige Rolle bei der Bildung der Kollagenfasern in der Haut.

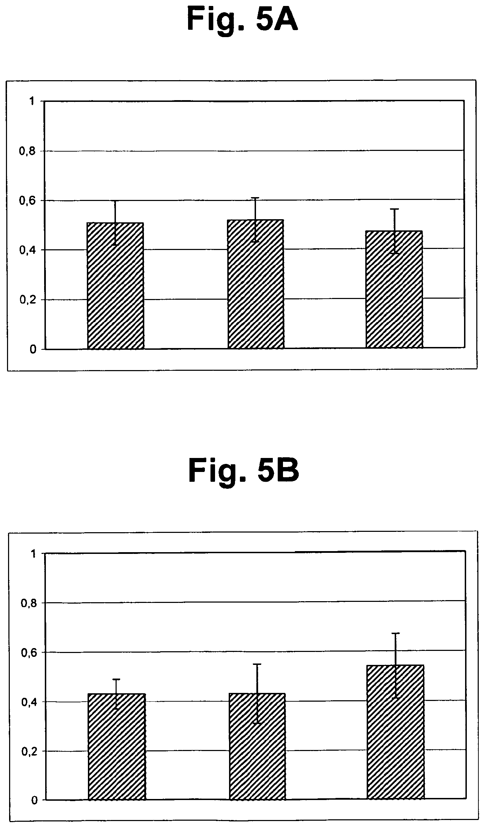

Die Ergebnisse sind als Säulendiagramme für das Hydrolysat B in der Figur 5A und für das Hydrolysat C in der Figur 5B dargestellt, wobei auf der Abszisse jeweils die RNA-Expression bei den kommerziellen Hydrolysaten B bzw. C relativ zur RNA-Expression bei dem erfindungsgemäßen Hydrolysat A (=1) aufgetragen ist. Die linke Säule steht jeweils für Typ-I-Kollagen, die mittlere Säule für Decorin und die rechte Säule für Versican. Es ist jeweils der Mittelwert aus mindestens 7 Messungen dargestellt sowie der Standardfehler.

Interessanterweise zeigen die Daten, dass bei allen drei Matrixproteinen eine im Vergleich zum Hydrolysat A deutlich geringere Stimulation der RNA-Synthese durch die beiden Hydrolysate B und C erfolgt, deren mittleres Molekulargewicht nur geringfügig höher ist. Die charakteristischen Peptide des Hydrolysats A scheinen somit eine entscheidende Rolle für dessen vorteilhafte Wirkung zu spielen.

### 8. Beispielrezepturen für Nahrungs(ergänzungs)mittel und Kosmetika

Im Folgenden sind einige beispielhafte Rezepturen für die Verwendung des Kollagenhydrolysats gemäß der Erfindung angegeben, die selbstverständlich in vielfältiger Weise abgewandelt werden können:

**Kapsetten (Nahrungsergänzungsmittel)**

| | |
|---|---|
| Glycerin | 53,67 Gew.% |
| **Kollagenhydrolysat** | 21,95 Gew.% |
| Gelatine | 10,08 Gew.% |
| Guarkernmehl | 6,00 Gew.% |
| Lecithin | 5,00 Gew.% |
| Citronensäure | 2,00 Gew.% |
| Aroma (Cassis) | 0,50 Gew.% |
| Orangenöl | 0,50 Gew.% |
| Acesulfam-K | 0,30 Gew.% |

**Schokolade**

| | |
|---|---|
| Kakaomasse | 51,0 Gew.% |
| Saccharose | 22,4 Gew.% |
| Kakaobutter | 16,6 Gew.% |
| **Kollagenhydrolysat** | 10,0 Gew.% |

**Getränk**

| | |
|---|---|
| Wasser | 63,00 Gew.% |
| Aloe-Vera-Konzentrat | 31,00 Gew.% |
| **Kollagenhydrolysat** | 4,00 Gew.% |
| Saccharose | 1,50 Gew.% |
| Citronensäure | 0,26 Gew.% |
| Aromen und Farbst. | 0,24 Gew.% |
| Sucralose | 0,0031 Gew.% |

**Haarshampoo**

| | |
|---|---|
| Wasser | 58,8 Gew.% |
| Natriumlaureth-11-carboxylat | 18,0 Gew.% |
| Cocoamidopropyl-Betain | 9,0 Gew.% |
| **Kollagenhydrolysat** | 6,0 Gew.% |
| PEG-6 Capryl/Caproyl-Glyceride | 3,0 Gew.% |
| PEG-150 Distearat | 2,5 Gew.% |
| Laureth-7 | 2,0 Gew.% |
| Kaliumsorbat | 0,5 Gew.% |
| Parfum | 0,2 Gew.% |

## Patentansprüche

1. Nicht-therapeutische Verwendung von Kollagenhydrolysat zur Stimulation der Biosynthese extrazellulärer Matrixproteine durch Hautzellen, zur Erhöhung der Spannkraft der Haut, zur Verminderung der Faltenbildung und/oder zur Erhöhung des Feuchtigkeitsgehaltes der Haut,
**dadurch gekennzeichnet,**
**dass** das Kollagenhydrolysat durch die nacheinander folgende Einwirkung von zwei Endoproteasen mit einer unterschiedlichen Spezifität bei 50 bis 60 °C während 120 bis 180 min hergestellt ist, wobei als erstes Enzym eine Endoprotease aus *Bacillus subtilis* oder aus *Bacillus amyloliquefaciens* verwendet wird und als zweites Enzym eine Endoprotease aus *Bacillus licheniformis,* dass mindestens 45 Gew.% des Kollagenhydrolysats ein Molekulargewicht von weniger als 1.500 Da aufweisen, und
**dass** das Kollagenhydrolysat mindestens vier charakteristische Peptide mit einem Molekulargewicht zwischen 600 und 1.200 Da umfasst, die in einer mittels MALDI-Massenspektroskopie bestimmten Molekulargewichtsverteilung eine mindestens doppelte Intensität im Vergleich zu ihrer Umgebung aufweisen, wobei das Massenspektrum unter Verwendung eines TOF/TOF-Massenspektrometers bestimmt wurde mit einer Probe des Kollagenhydrolysats, die in 0,1%-iger Trifluoressigsäure auf eine Endkonzentration von 10 µg/µl eingestellt, unter Verwendung von µC₁₈-Material gereinigt und mit einer HCCA-Matrix auf ein MALDI-Target präpariert wurde.

2. Verwendung nach Anspruch 1, wobei die mindestens vier charakteristischen Peptide in der mittels MALDI-Massenspektroskopie bestimmten Molekulargewichtsverteilung eine mindestens vierfache Intensität im Vergleich zu ihrer Umgebung aufweisen.

3. Verwendung nach einem der vorangehenden Ansprüche, wobei das Kollagenhydrolysat ein Peptid zwischen 620 und 690 Da, ein Peptid zwischen 790 und 860 Da, ein Peptid zwischen 980 und 1.050 Da und ein Peptid zwischen 1.175 und 1.245 Da umfasst.

4. Verwendung nach einem der vorangehenden Ansprüche, wobei das Kollagenhydrolysat weitere charakteristische Peptide mit einem Molekulargewicht zwischen 1.500 und 3.500 Da umfasst.

5. Verwendung nach einem der vorangehenden Ansprüche, wobei das Kollagenhydrolysat einen Anteil an Hydroxyprolin von 12 Gew.% oder mehr aufweist.

6. Verwendung nach einem der vorangehenden Ansprüche, wobei mindestens 50% der N-terminalen Aminosäuren des Kollagenhydrolysats hydrophobe Aminosäuren sind, insbesondere Alanin, Leucin und Isoleucin.

7. Verwendung nach einem der vorangehenden Ansprüche, wobei das Kollagenhydrolysat für eine orale Aufnahme vorgesehen ist, und insbesondere ein Nahrungsergänzungsmittel ist oder in einem Nahrungs- oder Genussmittel enthalten ist.

8. Verwendung nach Anspruch 7, wobei eine tägliche Aufnahme von ca. 1,5 bis 5 g, bevorzugt ca. 2 bis 3 g, weiter bevorzugt ca. 2,3 bis 2,7 g des Kollagenhydrolysats vorgesehen ist.

9. Verwendung nach einem der Ansprüche 1 bis 6, wobei das Kollagenhydrolysat für eine topische Anwendung vorgesehen ist.

10. Verwendung nach Anspruch 9, wobei das Kollagenhydrolysat in einer Creme, einer Salbe, einer Lotion, einem Gel oder einem Shampoo enthalten ist.

11. Kollagenhydrolysat zur Anwendung in einem Verfahren zur Verbesserung der Barrierefunktion der Haut,
**dadurch gekennzeichnet,**
**dass** dass das Kollagenhydrolysat durch die nacheinander folgende Einwirkung von zwei Endoproteasen mit einer unterschiedlichen Spezifität bei 50 bis 60 °C während 120 bis 180 min hergestellt ist, wobei als erstes Enzym eine Endoprotease aus *Bacillus subtilis* oder aus *Bacillus amyloliquefaciens* verwendet wird und als zweites Enzym eine Endoprotease aus *Bacillus licheniformis,* dass mindestens 45 Gew.% des Kollagenhydrolysats ein Molekulargewicht von weniger als 1.500 Da aufweisen, und dass das Kollagenhydrolysat mindestens vier charakteristische Peptide mit einem Molekulargewicht zwischen 600 und 1.200 Da umfasst, die in einer mittels MALDI-Massenspektroskopie bestimmten Molekulargewichtsverteilung eine mindestens doppelte Intensität im Vergleich zu ihrer Umgebung aufweisen, wobei das Massenspektrum unter Verwendung eines TOF/TOF-Massenspektrometers bestimmt wurde mit einer Probe des Kollagenhydrolysats, die in 0,1%-iger Trifluoressigsäure auf eine Endkonzentration von 10 µg/µl eingestellt, unter Verwendung von µC₁₈-Material gereinigt und mit einer HCCA-Matrix auf ein MALDI-Target präpariert wurde.

## Claims

1. Non-therapeutic use of collagen hydrolysate for stimulating the biosynthesis of extracellular matrix proteins by skin cells, for increasing the tonicity of the skin, for reducing the formation of wrinkles and/or for increasing the moisture content of the skin,
**characterized in that**
the collagen hydrolysate is produced by the consecutive action of at least two endoproteases with a different specificity at 50 to 60 °C for 120 to 180 min, wherein an endoprotease of *Bacillus subtilis* or of *Bacillus amyloliquefaciens* is used as the first enzyme and an endoprotease of *Bacillus licheniformis* is used as the second enzyme,
at least 45 wt.% of the collagen hydrolysate has a molecular weight of less than 1,500 Da, and
the collagen hydrolysate comprises at least four characteristic peptides with a molecular weight between 600 and 1,200 Da, which have at least twice the intensity in comparison to their surroundings in a molecular weight distribution determined by means of MALDI mass spectroscopy, wherein the mass spectrum has been determined using a TOF/TOF mass spectrometer with a sample of the collagen hydrolysate having been adjusted in 0.1% trifluoroacetic acid to a final concentration of 10 µg/µl, purified using µC₁₈ material and prepared with an HCCA matrix on a MALDI target.

2. Use according to claim 1, wherein the at least four characteristic peptides have at least four times the intensity in comparison to their surroundings in a molecular weight distribution determined by means of MALDI mass spectroscopy.

3. Use according to any one of the preceding claims, wherein the collagen hydrolysate comprises a peptide between 620 and 690 Da, a peptide between 790 and 860 Da, a peptide between 980 and 1,050 Da and a peptide between 1,175 and 1,245 Da.

4. Use according to any one of the preceding claims, wherein the collagen hydrolysate comprises further characteristic peptides with a molecular weight between 1,500 and 3,500 Da.

5. Use according to any one of the preceding claims, wherein the collagen hydrolysate has a hydroxyproline content of 12 wt.% or more.

6. Use according to any one of the preceding claims, wherein at least 50% of the N-terminal amino acids of the collagen hydrolysate are hydrophobic amino acids, in particular alanine, leucine and isoleucine.

7. Use according to any one of the preceding claims, wherein the collagen hydrolysate is provided for oral intake, and in particular is a nutritional supplement or is contained in a food product or luxury food product.

8. Use according to claim 7, wherein a daily intake of about 1.5 to 5 g, preferably about 2 to 3 g, more preferably about 2.3 to 2.7 g of the collagen hydrolysate is provided.

9. Use according to any one of claims 1 to 6, wherein the collagen hydrolysate is provided for topical application.

10. Use according to claim 9, wherein the collagen hydrolysate is contained in a cream, an ointment, a lotion, a gel or a shampoo.

11. Collagen hydrolysate for use in a method for improving the barrier function of the skin,
**characterized in that**
the collagen hydrolysate is produced by the consecutive action of at least two endoproteases with a different specificity at 50 to 60 °C for 120 to 180 min, wherein an endoprotease of *Bacillus subtilis* or of *Bacillus amyloliquefaciens* is used as the first enzyme and an endoprotease of *Bacillus licheniformis* is used as the second enzyme,
at least 45 wt.% of the collagen hydrolysate has a molecular weight of less than 1,500 Da, and
the collagen hydrolysate comprises at least four characteristic peptides with a molecular weight between 600 and 1,200 Da, which have at least twice the intensity in comparison to their surroundings in a molecular weight distribution determined by means of MALDI mass spectroscopy, wherein the mass spectrum has been determined using a TOF/TOF mass spectrometer with a sample of the collagen hydrolysate having been adjusted in 0.1% trifluoroacetic acid to a final concentration of 10 µg/µl, purified using µC₁₈ material and prepared with an HCCA matrix on a MALDI target.

## Revendications

1. Utilisation non thérapeutique d'hydrolysat de collagène pour stimuler la biosynthèse de protéines matricielles extracellulaires par des cellules cutanées, augmenter l'élasticité de la peau, réduire la formation des rides et/ou augmenter l'hydratation de la peau, **caractérisée en ce que** l'hydrolysat de collagène est fabriqué par l'action consécutive de deux endoprotéases ayant une spécificité différente à une température de 50 à 60°C pendant 120 à 180 minutes, la première enzyme utilisée étant une endoprotéase de *Bacillus subtilis* ou de *Bacillus amyloliquefaciens* et la deuxième enzyme une endoprotéase de *Bacillus licheniformis,* **en ce qu'**au moins 45 % en poids de l'hydrolysat de collagène a un poids moléculaire inférieur à 1 500 Da et **en ce que** l'hydrolysat de collagène comprend au moins quatre peptides caractéristiques ayant un poids moléculaire compris entre 600 et 1 200 Da qui présentent, dans une distribution des poids moléculaires, déterminée par spectroscopie de masse MALDI, une intensité au moins multipliée par deux par rapport à leur environnement, le spectre de masse ayant été déterminé avec un spectromètre de masse TOF/TOF avec un échantillon d'hydrolysat de collagène ajusté sur une concentration finale de 10 µg/µl dans de l'acide trifluoroacétique à 0,1 %, nettoyé en utilisant du matériel µC₁₈ et préparé avec une matrice HCCA sur une plaque cible MALDI.

2. Utilisation selon la revendication 1, dans laquelle les au moins quatre peptides caractéristiques présentent, dans la distribution des poids moléculaires déterminée par spectroscopie de masse MALDI, une intensité au moins multipliée par quatre par rapport à leur environnement.

3. Utilisation selon l'une quelconque des revendications précédentes, dans laquelle l'hydrolysat de collagène comprend un peptide entre 620 et 690 Da, un peptide entre 790 et 860 Da, un peptide entre 980 et 1 050 Da et un peptide entre 1 175 et 1 245 Da.

4. Utilisation selon l'une quelconque des revendications précédentes, dans laquelle l'hydrolysat de collagène comprend d'autres peptides caractéristiques ayant un poids moléculaire entre 1 500 et 3 500 Da.

5. Utilisation selon l'une quelconque des revendications précédentes, dans laquelle l'hydrolysat de collagène présente une proportion d'hydroxyproline de 12 % en poids ou plus.

6. Utilisation selon l'une quelconque des revendications précédentes, dans laquelle au moins 50 % des acides aminés N-terminaux de l'hydrolysat de collagène sont des acides aminés hydrophobes, en particulier de l'alanine, de la leucine et de l'isoleucine.

7. Utilisation selon l'une quelconque des revendications précédentes, dans laquelle l'hydrolysat de collagène est prévu pour être absorbé par voie orale et, en particulier, est un complément alimentaire ou est contenu dans un aliment ou un aliment d'agrément.

8. Utilisation selon la revendication 7, dans laquelle il est prévu une absorption quotidienne d'environ 1,5 à 5 g, de préférence d'environ 2 à 3 g, de manière davantage préférée d'environ 2,3 à 2,7 g d'hydrolysat de collagène.

9. Utilisation selon l'une quelconque des revendications 1 à 6, dans laquelle l'hydrolysat de collagène est prévu pour une utilisation topique.

10. Utilisation selon la revendication 9, dans laquelle l'hydrolysat de collagène est contenu dans une crème, une pommade, une lotion, un gel ou un shampoing.

11. Hydrolysat de collagène destiné à être utilisé dans un procédé d'amélioration de la fonction barrière de la peau, **caractérisé en ce qu'**il est fabriqué par l'action consécutive de deux endoprotéases ayant une spécificité différente à une température de 50 à 60°C pendant 120 à 180 minutes, la première enzyme utilisée étant une endoprotéase de *Bacillus subtilis* ou de *Bacillus amyloliquefaciens* et la deuxième enzyme une endoprotéase de *Bacillus licheniformis,* **en ce qu'**au moins 45 % en poids de l'hydrolysat de collagène a un poids moléculaire inférieur à 1 500 Da et **en ce que** l'hydrolysat de collagène comprend au moins quatre peptides caractéristiques ayant un poids moléculaire compris entre 600 et 1 200 Da qui présentent, dans une distribution des poids moléculaires déterminée par spectroscopie de masse MALDI, une intensité au moins multipliée par deux par rapport à leur environnement, le spectre de masse ayant été déterminé avec un spectromètre de masse TOF/TOF avec un échantillon d'hydrolysat de collagène ajusté sur une concentration finale de 10 µg/µl dans de l'acide trifluoroacétique à 0,1 %, nettoyé en utilisant du matériel µC₁₈ et préparé avec une matrice HCCA sur une plaque cible MALDI.
